# FASCICULE DE BREVET EUROPEEN

(11) **EP 1 682 158 B1**
(45) Date de publication et mention de la délivrance du brevet: **25.05.2011**
(21) Numéro de dépôt: 04805348.2
(22) Date de dépôt: 29.10.2004
(51) Int. Cl.: A61K 31/7004

(54) **Compositions cosmétiques comprenant au moins un monomère de rhamnose pour le traitement cosmétiques des peaux**
Kosmetische Zusammensetzung enthaltend mindestens ein Rhamnosemonomer zur kosmetische Behandlung der Haut
Cosmetic compositions comprising at least a rhamnose monomer for the cosmetic treatment of skins

(30) Priorité: 31.10.2003 FR 0312798
(43) Date de publication de la demande: 26.07.2006
(60) Demande divisionnaire: 10178389.2
(73) Titulaire: Pierre Fabre Dermo-Cosmétique, 92100 Boulogne-Billancourt (FR); CENTRE NATIONAL DE LA RECHERCHE SCIENTIFIQUE (CNRS), 75016 Paris (FR)
(72) Inventeur: HOULMONT, Jean-Philippe, 31520 Ramonville Saint Agne (FR); RICO-LATTES, Isabelle, 31650 Auzielle (FR); PEREZ, Emile, 31770 Colomiers (FR); BORDAT, Pascal, 31320 Mervilla (FR)
(74) Mandataire: Warcoin, Jacques
(86) Numéro de dépôt international: PCT/FR2004/002794
(87) Numéro de publication internationale: WO 2005/041983

(56) Documents cités:
- DE-A1- 19 704 693
- FR-A- 2 652 742
- FR-A- 2 756 735
- FR-A- 2 768 623
- FR-A- 2 813 789
- M. SCHMIDT, S.K. CHATTERJEE, B. DOBNER, P. NUHN: "New modified single chained glycolipids" CHEMISTRY AND PHYSICS OF LIPIDS, vol. 114, 2002, pages 139-147, XP002282380

## Description

La présente invention concerne de nouvelles compositions comprenant au moins un monomère de rhamnose pour le traitement cosmétique des peaux et/ou des muqueuses sensibles, irritées, intolérantes, à tendance allergique, âgées, soumises à un signal danger, présentant un trouble de la barrière cutanée, présentant des rougeurs cutanée ou présentant un déséquilibre immunologique non pathologique lié au vieillissement intrinsèque, extrinsèque ou hormonal, pour retarder le vieillissement naturel de la peau et/ou pour prévenir le vieillissement accéléré de la peau.
La réaction inflammatoire est une réponse du système immunitaire face à une agression causée aux cellules ou aux tissus vascularisés d'un organisme par un pathogène tel que les virus, les bactéries ou bien face à une agression chimique ou physique. Souvent douloureuse, l'inflammation est généralement une réponse de guérison. Cependant dans certains cas, (arthrite rhumatoïde, la maladie de Crohn, les maladies autoimmunes...) elle peut avoir des conséquences plus graves que le stimulus d'origine.
Les réactions d'hypersensibilité de contact correspondent à des réactions d'immunité spécifique dirigées contre des antigènes localisés sur des cellules ou dans les tissus, à l'origine de lésions cellulaires ou de réactions inflammatoires. Ces réactions d'hypersensibilité peuvent se développer dans le cadre des mécanismes de défense vis-à-vis d'un microorganisme pathogène ou dans le cas de réactions allergiques. Elles font intervenir différents types de cellules et en particulier les cellules de la peau, certains leucocytes, sans oublier les cellules endothéliales dont le rôle est prépondérant dans les réactions inflammatoires.
Les interactions intercellulaires qui interviennent alors impliquent généralement des phénomènes de reconnaissances spécifiques entre ligands et récepteurs. Au cours de ces vingt dernières années, de nombreux récepteurs de surface cellulaires ont été identifiés, tels que des protéines capables d'assurer une reconnaissance spécifique avec certains sucres comme le fucose et le rhamnose.

Les lectines sont des protéines implantées dans les membranes des cellules eucaryotes, et jouent un rôle très important dans les phénomènes d'adhésion et de reconnaissance entre cellules notamment lors des processus inflammatoires. Les lectines membranaires sont impliquées en particulier dans l'endocytose, le trafic intracellulaire des glycoconjugués et la perméabilité endothéliale. De plus, ces protéines, souvent transmembranaires, contribuent à la reconnaissance spécifique de l'antigène (domaine extracellulaire) et à l'activation des cellules (domaine intracellulaire). Les lectines peuvent reconnaître spécifiquement certains sucres, notamment le rhamnose.
Depuis de nombreuses années, les alkyles polysaccharides (CmGn où m est le nombre de carbones dans la chaîne alkyle et n le nombre d'unités glycosidiques composant la tête hydrophile) constituent une intéressante famille de tensioactifs non ioniques. Par exemple, les alkyles polyglucosides (APGs) qui sont préparés industriellement à partir du glucose et d'alcool gras, trouvent de nombreuses applications en détergence mais également en cosmétologie en raison de leur bonne tolérance dermatologique.
La publication « cosmetic use formulation containing pentyl rhamnoside and cetyl rhamnoside », J P Houlmont and al, International Journal of Cosmetic Science, 2001, 23, 363-368, décrit la synthèse et l'utilisation du pentyle et cétyle rhamnoside comme co-tensioactif et tensioactif respectivement, ainsi que leur adéquation pour la formulation en cosmétique. Ces alkyles rhamnosides (en C₅ et C₁₆) sont produits directement par acétalisation du L-rhamnose dans un alcool approprié en présence d'un catalyseur acide. Ces alkyles rhamnosides sont décrits comme étant biocompatibles et peu toxiques.
La demande de brevet EP 0 804 923 décrit une composition comprenant un alkyle éther polysaccharide qui comprend au moins deux unités sucres différentes et au moins un groupe hydroxyle substitué par une chaîne alkyle saturée en C₁-C₂₄. Cette composition permet de protéger la peau envers les rayonnements ultraviolets.
La demande de brevet EP 0 804 924 décrit une composition destinée à prolonger la longévité d'un parfum sur la peau qui comprend au moins un alkyle d'éther polysaccharide comprenant au moins deux unités sucres différentes et au moins un groupe hydroxyle substitué par une chaîne alkyle en C₁-C₂₄ saturée.

La demande de brevet FR 2 756 735 décrit des compositions pharmaceutiques ou cosmétiques comprenant au moins un composé fucosique ou rhamnosique, en particulier le α-L-fucose ou le α-L-rhamnose. Le composé peut être associé ou combiné à un reste lipidique ou protéique. Le composé est un agent d'inhibition de l'expression de la protéine d'adhérence ICAM-1 par les kératinocytes associés.

La demande de brevet FR 2 768 623 décrit des compositions cosmétiques et/ou dermatologiques à usage topique pour le traitement des peaux grasses pouvant comprendre du rhamnose (complexe anti-inflammatoire).

La demande de brevet FR 2 652 742 décrit des compositions anallergiques cosmétiques et dermo-pharmaceutiques pouvant comprendre le L-rhamnose.

La présente invention concerne une composition comprenant au moins un monomères d'alkyl-rhamnose de formule I : 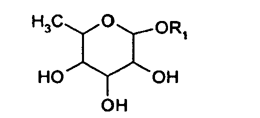 dans laquelle R₁ représente un radical alkyle en C₅-C₁₂ pour le traitement cosmétique des peaux et/ou des muqueuses sensibles, irritées, intolérantes, à tendance allergique, âgées, soumises à un signal danger, présentant un trouble de la barrière cutanée, présentant des rougeurs cutanée ou présentant un déséquilibre immunologique non pathologique lié au vieillissement intrinsèque, extrinsèque ou hormonal.

R₁ représente avantageusement un radical alkyle en C₅-C₈. Selon une variante avantageuse de l'invention, R₁ représente un radical choisi dans le groupe constitué par le pentyle, l'octyle, le décyle et l'undécyle.
Dans le cadre de la présente invention, on parlera indifféremment de monomère d'alkyl-rhamnose, ou d'alkyl-rhamnoside.
Le rhamnose peut être de configuration lévogyre ou dextrogyre. Selon une variante avantageuse de l'invention, le rhamnose est de configuration lévogyre.
Le rhamnose peut être sous la forme anomérique α ou β. Selon une autre variante avantageuse de l'invention, le rhamnose est sous la forme anomérique α.

Dans le cadre de la présente invention, les monomères de sucres réducteurs dont une fonction hydroxyle est substituée par un radical alkoxy sont dénommés des alkyl-sucres réducteurs. Les alkyl-sucres réducteurs répondent à la formule générale suivante : 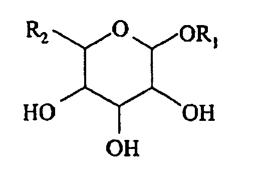 dans laquelle R₂ représente le radical -CH₃ ou le radical -CH₂OH et R₁ représente un radical alkyle en C₅-C₁₂.
Au sens de la présente invention, on entend par « sucre réducteur » un sucre présentant, lorsqu'il est sous forme linéaire, une fonction aldéhyde libre portée par le carbone anomère. Les sucres réducteurs peuvent être mis en évidence dans une solution avec un test à la liqueur de Fehling. Comme exemple de sucres réducteurs, on peut notamment citer le glucose, le fructose, le maltose, le galactose et le lactose.
Dans le cadre de la présente invention, le sucre réducteur est le rhamnose.
Le rhamnose peut être de configuration lévogyre ou dextrogyre. Selon une variante avantageuse de l'invention, le rhamnose, est de configuration lévogyre.
Le rhamnose peut être sous la forme anomérique α ou β. Selon une autre variante avantageuse de l'invention, le rhamnose, est sous la forme anomérique α.
Selon une variante avantageuse de l'invention, le radical alkoxy comprend de 5 à 8 atomes de carbone. Ainsi R₁ représente avantageusement un radical alkyle en C₅-C₁₂, de préférence en C₅-C₈. Selon une variante avantageuse de l'invention, R₁ représente un radical choisi dans le groupe constitué par le pentyle, l'octyle, le décyle et l'undécyle.
L'invention est caractérisée par le fait que le sucre réducteur, portant un radical alkyl, est uniquement sous forme monomérique.

Lesdits monomères d'alkyl-rhamnose selon l'invention, peuvent être synthétisés en une seule étape réactionnelle, sans aucune étape de protection ou de déprotection des fonctions hydroxyles du sucre réducteur, par réaction de condensation du rhamnose, avec un alcool, ayant un nombre d'atomes correspondant à la longueur de la chaîne alkyle.
Le procédé de synthèse utilisé est une réaction type réaction de Fischer avec l'acide *p-*toluène sulfonique (APTS) comme catalyseur acide. Elle s'effectue de manière « one-pot », les réactifs (sucre réducteur : en particulier rhamnose, alcool, et catalyseur acide) sont mis en présence les uns avec les autres sans utiliser de solvant, le milieu est alors sous forme hétérogène.
Le mélange rhamnose alcool et catalyseur acide est avantageusement mis à réagir sous chauffage et éventuellement sous agitation à une température comprise entre 20 et 120°C, encore plus avantageusement entre 35 et 75°C. La température ne doit pas être trop élevée, notamment elle ne doit pas dépasser 120°C, afin d'éviter une dégradation des sucres. Le mélange est avantageusement mélangé de 5 minutes à 24 heures, encore plus avantageusement 3 heures.
La fonction anomérique étant la plus réactive, grâce à la stabilisation du carbocation par résonance, l'addition de l'alcool se fait uniquement en position 1.
L'alcool, en excès, dans un rapport molaire environ double par rapport au rhamnose, sert de solvant au produit de synthèse, le milieu réactionnel se trouve donc en phase homogène en fin de réaction. On choisit comme catalyseur acide un acide de Brönsted relativement fort, mais soluble en milieu organique, tel que l'APTS. L'acide sulfurique trop puissant et l'acide chlorhydrique n'ont donc pas pu être utilisés car ils sont solubles dans l'eau tandis que les acides carboxyliques n'étaient pas suffisamment forts. Il faut éviter de travailler en présence d'eau, car elle favoriserait davantage la réaction inverse d'hydrolyse plutôt que la réaction d'addition.
L'auto-condensation des monomères de sucres réducteurs, en particulier d'alkyl-rhamnose, d'alkyl-fucose ou d'alkyl-glucose, est limitée voire supprimée, bien que chaque fonction hydroxyle, d'un point de vue théorique, est à même de réagir avec une autre pour créer une liaison glycosidique et donc d'augmenter le degré de polymérisation, en raison de l'absence d'agents protecteurs. On suppose que cette auto-condensation est supprimée du fait que les 6-déoxy-sucres (rhamnose, fucose en particulier) sont dépourvus de fonction hydroxyle primaire et possèdent un groupement méthyle à la place. Le groupement méthyle des 6-déoxy sucres favoriserait la formation d'alkyl-monosaccharides en supprimant une fonction hydroxyle très réactive portée par le carbone 6 et en ajoutant une gêne stérique au voisinage du carbone 4
Cette synthèse conduit pour l'ensemble des alkyl-sucres réducteurs à l'anomère α, configuration où la gêne stérique est minimisée et donc la plus stable thermodynamiquement. En particulier, cette synthèse conduit, pour la majorité des alkyl-rhamnosides à l'anomère α. Le rapport anomérique α/β pour les alkyl-fucosides est voisin de 2.
Selon une variante avantageuse de l'invention, l'eau formée pendant la réaction de condensation est éliminée, physiquement ou chimiquement. Comme exemple de technique d'élimination physique de l'eau formée pendant la synthèse, on peut notamment citer la distillation ou l'utilisation d'un adsorbant. Comme exemple de technique d'élimination chimique de l'eau formée pendant la synthèse, on peut notamment citer l'utilisation d'un agent de dessiccation.
L'eau formée pendant la réaction de condensation est avantageusement éliminée au moyen d'un agent de dessiccation choisi dans le groupe constitué par les carbonates, les sulfates, le chlorure de calcium, le pentaoxyde de phosphore, des tamis moléculaires ou des combinaisons de ces divers agents de dessiccation. L'agent de dessiccation peut être introduit directement dans le milieu réactionnel.
Selon une variante de l'invention, la réaction de condensation est réalisée à pression atmosphérique et sous atmosphère d'un gaz inerte, tel que l'argon ou l'azote.

Selon une autre variante de l'invention, la réaction de condensation est réalisée à pression réduite.
Selon une variante avantageuse de l'invention, à la fin de la réaction de condensation, le mélange est amené à plus basse température, de quelques degrés sous la température de réaction à 0°C, de préférence à température ambiante, et repris dans un solvant capable de solubiliser le monomère d'alkyl-rhamnose, ledit solvant est avantageusement le dichlorométhane. Le catalyseur acide est neutralisé à l'aide d'une base faible, de préférence l'hydrogénocarbonate, pendant une période allant de 1 minute à 24 heures, avantageusement 30 minutes.
Selon les différentes longueurs de chaîne, les produits sont purifiés, soit par chromatographie sur colonne pour les chaînes les plus courtes, soit par soxlhet pour les autres composés. Les deux méthodes peuvent être combinées si l'on recherche une très grande pureté.
Le principe de la purification par soxhlet consiste à mélanger le brut de la réaction (alkyl-rhamnoside, alcool résiduel, APTS, rhamnose) avec de la silice pour chromatographie dans des rapports massiques voisins de 1 :4, et de placer ce mélange dans une cartouche d'extraction : couplage d'une méthode d'extraction solide-liquide à chaud avec une méthode de chromatographie en continu.
Le rendement massique de ce procédé de synthèse de monomères d'alkyl-rhamnose est supérieur à 40%.
La composition selon l'invention est avantageusement destinée à réguler les mécanismes inflammatoires.

La composition est notamment destinée à la prévention ou au traitement des réactions ou pathologies allergiques, inflammatoires, immunitaires de la peau et/ou des muqueuses. La composition selon l'invention est également destinée à inhiber la réponse immune liée au stress inflammatoire.
La composition selon l'invention est notamment destinée à inhiber l'activation des leucocytes, tels que les polynucléaires humains et notamment les neutrophiles et les mastocytes humains empêchant la libération des médiateurs préformés de la réaction immunitaire. Elle permet également l'inhibition de l'adhésion des lymphocytes circulants et des cellules endothéliales empêchant ainsi la transmigration de ces leucocytes sur le lieu de l'inflammation. Elle permet aussi l'inhibition de la sécrétion des cytokines kératinocytaires, activatrices des lymphocytes T et des cellules de Langerhans, telles que IL-1, TNF-α, ou des molécules d'adhésion, telles que ICAM-1, VCAM, qui contribuent au recrutement et au passage trans-endothélial des leucocytes. La composition selon l'invention est également inhibitrice du phénomène d'hyperplasie kératinocytaire.
La composition selon l'invention est aussi inhibitrice du processus d'apprêtement de l'antigène par les cellules dendritiques de la peau, de la maturation des cellules présentatrices d'antigène, à savoir les cellules dendritiques dermiques et les cellules de Langerhans, du phénomène de reconnaissance entre les lymphocytes et les cellules présentatrices d'antigène.
Ainsi, la composition selon l'invention est destinée à la prévention ou au traitement des maladies choisies dans le groupe constitué par de l'eczéma atopique et/ou de contact, des dermatoses inflammatoires, des dermatites irritatives, de l'acné, des maladies auto-immunes telles que le psoriasis, de la photo-immuno-suppression, du vitiligo, du pityriasis, des sclérodermies, de l'arthrite rhumatoïde, de la maladie de Crohn ou du rejet de greffe.
La composition selon l'invention est également destinée à la prévention et au traitement des dérives inflammatoires chroniques liées au vieillissement et ses conséquences. La composition est notamment destinée à la prévention ou au traitement des maladies choisies dans le groupe constitué par les sensibilisations anaphylactiques, les anomalies pigmentaires de la peau, l'hypervascularisation dermique, les fissurations inflammatoires.
Selon une variante de l'invention, la composition est destinée à diminuer le caractère allergisant et/ou irritant d'une composition ou d'un parfum. La composition selon l'invention comprend avantageusement 0,001 à 50% en poids d'alkyl-sucres réducteurs.
La composition selon la présente invention peut être formulée pour être administré par toute voie. Elle est avantageusement formulée pour être administrée par voie topique, orale, sous-cutanée, injectable, rectale et génitale.
Lorsque la composition est formulée pour être administrée par voie orale, ladite composition peut se présenter sous la forme de solution aqueuse, émulsion, comprimés, gélules, capsules, poudres, granules, solutions ou encore de suspensions orales.
Lorsque la composition est formulée pour être administrée par voie sous-cutanée, ladite composition peut se présenter sous la forme d'ampoules injectables stériles.

Lorsque la composition est formulée pour être administrée par voie rectale, ladite composition peut se présenter sous la forme de suppositoires.
Lorsque la composition est formulée pour être administrée par voie génitale, ladite composition peut se présenter sous la forme d'ovules.
Ladite composition selon l'invention est de préférence à application topique. Ladite composition peut alors être formulée de manière à se présenter par exemple sous forme de solution aqueuse, de crème blanche ou colorée, de pommade, de lait, de lotion, de gel, d'onguent, de sérum, de pâte, de mousse, d'aérosol ou de stick.
La quantité de la composition selon l'invention à administrer dépend de la gravité et de l'ancienneté de l'affection traitée. Naturellement, le médecin adaptera aussi la posologie en fonction du malade.

La présente invention concerne également une composition comprenant au moins un monomère de rhamnose, dont la fonction hydroxyle anomérique est substituée par un radical alkoxy en C₅-C₁₂ pour retarder le vieillissement naturel de la peau et/ou pour prévenir le vieillissement accéléré de la peau soumise aux agressions extérieures, notamment pour prévenir le vieillissement photo-induit de la peau.

La composition cosmétique appliquée dans la méthode de traitement cosmétique selon l'invention comprend avantageusement 0,001 à 50% en poids de rhamnose.
Le rhamnose peut être de configuration lévogyre ou dextrogyre. Selon une variante avantageuse de l'invention, le rhamnose est de configuration lévogyre.
Le rhamnose peut être sous la forme anomérique α ou β. Selon une autre variante avantageuse de l'invention, le rhamnose, est sous la forme anomérique α.
Selon une variante avantageuse de l'invention, le radical alkoxy comprend de 5 à 8 atomes de carbone. Selon une variante avantageuse de l'invention, R₁ représente un radical choisi dans le groupe constitué par le pentyle, l'octyle, le décyle et l'undécyle.
Dans le cadre de l'invention, les alkyl-sucres réducteurs peuvent être préparés suivant le procédé décrit précédemment ou par tout autre procédé connu de l'homme du métier. Lorsque la composition cosmétique est formulée pour être administrée par voie topique, ladite composition se présente par exemple sous forme de solution aqueuse, de crème blanche ou colorée, de pommade, de lait, de lotion, de gel, d'onguent, de sérum, de pâte, de mousse, d'aérosol, de shampoing ou de stick.

D'autres caractéristiques et avantages de l'invention apparaissent dans la suite de la description avec les exemples représentés ci-après. Dans ces exemples on se référera aux figures suivantes. Ces figures et exemples sont destinés à illustrer la présente invention et ne peuvent en aucun cas être interprétés comme pouvant en limiter la portée.
- **Figure 1 :**: Viabilité des cellules endothéliales issues des ganglions lymphatiques périphériques en présence de rhamnose.
- **Figure 2 :**: Viabilité des cellules endothéliales issues des ganglions lymphatiques périphériques en présence de pentyle-rhamnoside.

### Exemple 1: Procédé de synthèse du dodécyl-rhamnoside

Dans un ballon à 2 voies de 100 mL, surmonté d'un réfrigérant droit lui-même équipé d'une garde desséchante (CaCl₂), on introduit sous balayage d'argon, 2 g de rhamnose (1 équivalent) et l'alcool gras (dodécyl alcool) à raison de 2 équivalents molaires (4,6 g). Le catalyseur acide *p*-toluène sulfonique (APTS) est ajouté au mélange hétérogène précédent maintenu sous argon à raison de 0,1 équivalent molaire. Le milieu est agité (agitateur magnétique) à 70°C pendant 3 heures.
Après réaction, le milieu devenu homogène, est refroidi à température ambiante. Une solution de dichlorométhane (20 mL) et une pointe de spatule de NaHCO₃ sont ajoutées au mélange laissé sous agitation et maintenu sous argon. Le milieu est ainsi abandonné pendant 30 minutes.
La solution est ensuite filtrée sur papier. Le filtrat (P2) contenant les alkyl-rhamnosides est évaporé et concentré en une huile visqueuse.
On récupère ainsi 1,9 g de P2 après filtration, et un rendement de 48 % après une étape de purification sur batch de silice.
La nature des alkyl-rhamnosides est déterminée par RMN, HPLC et spectrométrie de masse.
La spectrométrie de masse est réalisée par électrospray. Ces analyses font apparaître un degré de polymérisation maximal de 2 pour la tête polaire.
Les analyses RMN à 250 MHz se font dans le CDCl₃ ou D₂O sur un appareil multinoyaux Brücker AC250 fonctionnant à 250,13 MHz pour ¹H.
Les analyses chromatographiques sont réalisées en phase inverse sur une colonne greffée C18 (YMC-pack C18, 12 nm diamètre de pore moyen, 5 µm de diamètre des particules) et une colonne LiChrospher 100 RP-8 (125*4 mm I.D.). L'éluant choisi est un mélange acétonitrile-eau (40-60), avec un débit de 1,5 mL/min à 45 °C. Le détecteur est un détecteur à diffusion de la lumière Sedex 45.
Ces analyses permettent d'identifier l'alkyl-rhamnoside présent dans P2 qui est le dodécyl-rhamnoside.

Le pentyl-rhamnoside, l'octyl-rhamnoside, le décyl-rhamnoside et l'undécyl-rhamnoside peuvent être synthétisés suivant le même procédé en remplaçant respectivement le dodécyl alcool par le pentyl alcool, l'octyl alcool, le décyl alcool et l'undécyl alcool.

### Exemple 2 : réactivité des sucres vis à vis de l'alcool

Dans le cas des alcools linéaires à courte chaîne, la réactivité des différents sucres vis à vis de l'alcool est bonne. En effet, si on considère deux familles de composés, les alkyl-rhamnosides et les alkyl-glucosides, les résultats obtenus pour les pentyl-saccharides sont comparables. L'alcool, peu hydrophobe, assure un bon contact par une bonne mouillabilité du sucre et la réactivité est donc accrue. Les rendements obtenus pour les alcools de plus courte chaîne sont toujours supérieurs à 50 %.
Dans le cas particulier des alcools dont la longueur de chaîne est supérieure à 8 carbones, les rendements des alkyl-glucosides décroissent très rapidement quand la longueur de la chaîne hydrocarbonée augmente. L'alcool devient en effet trop hydrophobe et le contact avec le glucose très hydrophile est de moins en moins bien assuré.
Cette baisse de la réactivité est bien moins importante dans le cas du rhamnose (cf. tableau 1). Les rendements pour les alkyl-rhamnosides sont environ 8 fois supérieurs à ceux des alkyl-glucosides pour la chaîne en C₁₂ et encore presque 6 fois pour la chaîne en C₁₆.

**Tableau 1 : Rendements massiques obtenus après purification d'alkyl-glucosides et de β-alkyl-rhamnosides.**

| | | | |
|---|---|---|---|
| Rh-C₅ | 50 % | Glu-C₅ | 65 % |
| Rh-C₁₂ | 47 % | Glu-C₁₂ | 6 % |
| Rh-C₁₆ | 27 % | Glu-C₁₆ | 5 % |

Dans le tableau 1, l'abréviation Rh représente le rhamnose et l'abréviation Glu représente le glucose. Ainsi, par exemple, Rh-C₅ représente le pentyl-rhamnoside.

Une des raisons proposées pour expliquer cette réactivité est une plus grande hydrophobie des déoxy-sucres. En effet, la suppression de l'hydroxyle en position 6 et la présence du groupement méthyle permettent d'augmenter l'hydrophobie du sucre.

La présence du groupement méthyle sur le cinquième carbone permet également d'augmenter la densité électronique de l'oxygène par un effet inductif positif et ainsi stabiliser l'intermédiaire intervenant dans le mécanisme réactionnel. Le rhamnose est donc plus réactif que les hexoses vis à vis des alcools gras.

### Exemple 3 : Aspect physique des alkyl-rhamnosides en fonction de la longueur de chaîne du radical alkyle

Les alkyl-rhamnosides ayant des chaînes linéaires en C₅, C₆, C₈, C₁₀, C₁₁, et oléyle sont sous forme d'un liquide visqueux.
Les alkyl-rhamnosides ayant des chaînes linéaires en C₁₄, C₁₆, C₁₈ et C₂₂ sont sous forme solide.
L'alkyl-rhamnoside ayant une chaîne linéaire en C₁₂ est un gel très compact.

### Exemple 4: analyse pharmacologique des alkyl-rhamnosides

Les différentes cellules de l'immunité intervenant dans ces processus de l'inflammation ont été étudiées. Ce sont les cellules dendritiques de la peau, les cellules endothéliales, certains leucocytes et les kératinocytes.

### 1) Principes des techniques de mesure de la viabilité cellulaire

### ❖ technique de réduction du MTT, [3-(4,5-dimethyltiazol-2-yl)-2,5-diphenyl tetrazolium bromide] (commercialisé par Sigma)

Cette technique correspond à un test colorimétrique permettant la quantification des cellules vivantes, métaboliquement actives de manière non radioactive. Le MTT est une molécule cationique qui se fixe aux membranes des mitochondries de façon potentiel-dépendant. Au niveau des mitochondries le MTT sera réduit en bleu de formazan par la déshydrogénase mitochondriale. Les cellules vivantes se colorent donc en bleu, à l'inverse des cellules mortes qui restent transparentes. La mesure de la viabilité est ensuite réalisée par mesure de la densité optique à l'aide d'un lecteur automatique.

Toutefois cette méthode d'analyse semble être mieux adaptée aux cellules adhérentes (type kératinocytes) que pour les cellules non adhérentes (monocytes et cellules dendritiques). Une autre étude a donc été envisagée pour conclure sur la cytotoxité des oligorhamnosides vis-à-vis des cellules différenciées analysées : la cytométrie de flux en présence d'iodure de propidium.

### ❖ technique de réduction du sel de tétrazolium XTT.

Il s'agit d'une technique permettant une quantification de la prolifération cellulaire et du nombre de cellules vivantes, (métaboliquement actives) sans incorporation d'isotopes radioactifs. Le XTT, de couleur jaune, est une molécule cationique qui se fixe aux membranes des mitochondries de façon potentiel-dépendant, comme le fait le MTT.
Au niveau des mitochondries, le XTT sera réduit en formazan (orange) par la tétrazolium réductase mitochondriale. Cette méthode, plus onéreuse que la méthode du MTT, ne nécessite pas dans son protocole la lyse des cellules par le SDS pour libérer le colorant. En effet le produit de réduction est soluble au sein de la cellule. La méthode est ainsi plus rapide. Les cellules vivantes, en absence et en présence d'un traitement avec un produit se colorent, à l'inverse des cellules mortes qui restent incolores. Le taux de formazan produit est détecté au spectrophotomètre pour une longueur d'onde de 450 nm et est directement proportionnel au nombre de cellules métaboliquement actives.

### 2) tests de toxicité

❖ Des kératinocytes sont isolés et mis en culture à partir de biopsie de peau humaine issue de donneurs anonymes suite à une intervention de chirurgie plasmique. Les mesures de densité optique (absorbance) des 4 puits traités avec la même concentration de produits sont moyennées. Cette moyenne est comparée à la moyenne des mesures obtenues pour les 4 puits témoin (test t de Student -comparaison des moyennes-différence significative à 95 % si p<0,05 et à 99 % si p<0,01).
Les viabilités des cellules traitées sont exprimées en pourcentage par rapport au témoin (cellules non traitées) de 100 % (DO traité/ DO témoin × 100).

Le rhamnose ne présente pas de cytotoxicité (cf. tableau 2), même pour les concentrations les plus élevées.

**Tableau 2 : Viabilité des kératinocytes en présence de différentes concentrations en rhamnose.**

| | Témoin | Rhamnose 1 mg/mL | Rhamnose 0,1 mg/mL | Rhamnose 0,01 mg/mL | Rhamnose 0,001 mg/mL |
|---|---|---|---|---|---|
| % viabilité | 100 | 104 | 98 | 100 | 95 |
| p (Student) | | 0,504 | 0,679 | 0,991 | 0,407 |

Le pentyl-rhamnoside présente une cytotoxicité pour des concentrations supérieures à 2 mg/mL (cf. tableau 3). Cette toxicité ne peut pas être expliquée par un effet délipidant de l'alkyl-rhamnoside étant donné qu'à une concentration nettement supérieure, voisine de 70 g/L, aucun effet de détergence n'a été mis en évidence lors des études sur les vésicules multilamellaires de phosphatidylcholine.

**Tableau 3 : Viabilité des kératinocytes en présence de différentes concentrations en pentyl-rhamnoside (Rh-C₅).**

| | Témoin | Rh-C₅ 5 mg/mL | Rh-C₅ 2 mg/mL | Rh-C₅ 1 mg/mL | Rh-C₅ 0,1 mg/mL |
|---|---|---|---|---|---|
| % viabilité | 100 | 40 | 53 | 74 | 94 |
| p (Student) | | <0,01 | <0,01 | <0,01 | 0,145 |

Des tests de toxicité ont également été effectués avec l'undécyl-rhamnose et l'octadécyl-rhamnose. Les résultats sont donnés dans le tableau 4 suivant :

**Tableau 4 : Viabilité des kératinocytes en présence de différentes concentrations en C₁₁ et C₁₈ alkyl rhamnosides.**

| | Témoin | Rh-C₁₁ 500 µg/mL | Rh-C₁₁ 250 µg/mL | Rh-C₁₁ 100 µg/mL | Rh-C₁₁ 10 µg/mL |
|---|---|---|---|---|---|
| % viabilité | 100 | 30 | 11 | 17 | 81 |
| p (Student) | | <0,01 | <0,01 | <0,01 | <0,01 |
| | Témoin | Rh-C₁₈ 500 µg/mL | Rh-C₁₈ 250 µg/mL | Rh-C₁₈ 100 µg/mL | Rh-C₁₈ 10 µg/mL |
| % viabilité | 100 | 27 | 25 | 87 | 137 |
| p (Student) | | <0,01 | <0,01 | <0,05 | <0,01 |

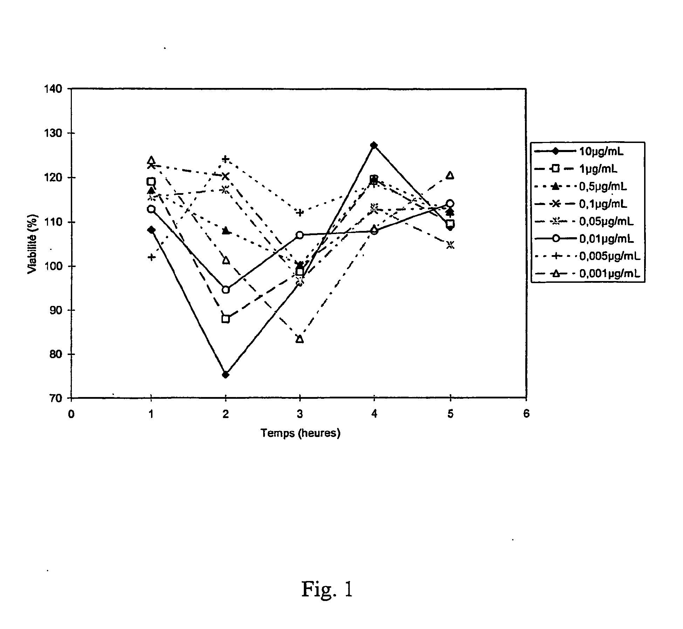

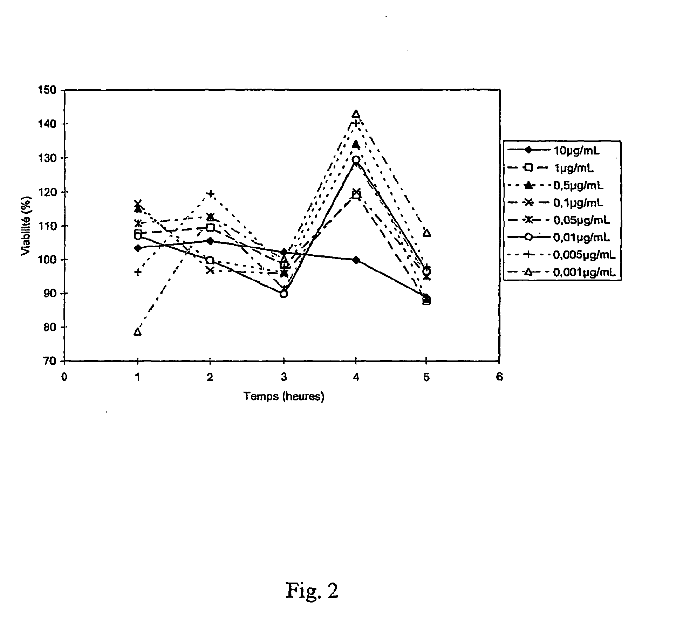

❖ Des cellules endothéliales ont été mises en culture, immortalisées et stabilisées dans leur phénotype. Les lignées cellulaires étudiées sont les cellules endothéliales d'appendice, les cellules endothéliales microvasculaire de cerveau, les cellules endothéliales de ganglions lymphatiques mésentérique, les cellules endothéliales de ganglions lymphatiques périphériques, les cellules endothéliales microvasculaires de la peau.
Le test de cytotoxicité est réalisé au moyen d'un test biochimique sur la transformation d'un sel de tétrazolium, le MTT. Les résultats obtenus sont très positifs, aucune toxicité n'est mise en évidence, avec le pentyl-rhamnoside (cf. figures 1 et 2). La viabilité est en effet toujours supérieure à 85 %, et ceci pour toutes les lignées de cellules étudiées.
- **Figure 1:**: Viabilité des cellules endothéliales issues des ganglions lymphatiques périphériques en présence de rhamnose.
- **Figure 2:**: Viabilité des cellules endothéliales issues des ganglions lymphatiques périphériques en présence de pentyl-rhamnoside.
On note en particulier l'apparition d'un pic de stimulation correspondant à 4 heures d'incubation, temps nécessaire pour l'initiation de la synthèse protéique. La présence de ce pic est intéressante car il traduit que les cellules tolèrent les oligorhamnosides (absence de toxicité), et les assimilent. Ces produits semblent enrichir le milieu de culture.
Ces résultats sont similaires pour les autres lignées de cellules endothéliales.

### 3) Influence des alkyl-rhamnosides sur des cellules humaines cultivées en milieu pro-inflammatoire

### • Dosage de la PGE₂ libérée par les KHN stimulées par le PMA.

Les alkyl-rhamnosides ont pu être évalués en tant qu'inhibiteur de la libération de PGE₂ dans les surnageants cellulaires. Ces produits sont mis en présence des KHN en même temps que le PMA à 1 ng/mL. Chaque condition testée est évaluée sur 4 puits de KHN pour la stimulation.
Les abréviations KHN signifient kératinocytes humains normaux.
Les abréviations PMA signifient phorbol-12-myristate-13-acétate.
Après 24 heures de traitement, les résultats résumés dans le tableau 5 suivant représentent les moyennes des valeurs des concentrations en PGE₂ (pg/mL) données dans chacun des surnageants cellulaires stimulés ou non et rapportées à une quantité de cellules exprimées en µg.

**Tableau 5 : Pourcentage d'inhibition de la libération de PGE₂ en fonction de la concentration des alkyl-rhamnosides (Rh-C₅, Rh-C₁₁ et Rh-C₁₈).**

| | 1 mg/mL | 0,5 mg/mL | 0,1 mg/mL | 0,05 mg/mL | 0,02 mg/mL | 0,01 mg/mL |
|---|---|---|---|---|---|---|
| Rh-C₅ | 89% | 84% 63% | 64% | 61% | 29% | - |
| Rh-C₁₁ | | | | | 28% | 28% |
| Rh-C₁₈ | | | | | 2% | |

Le pentyl-rhamnoside présente une plus forte inhibition (80 à 60 %) pour des concentrations de 1 mg/mL à 0,05 mg/mL. Son activité diminue à 0,02 mg/mL : 29 % d'inhibition.
Le pentyl-rhamnoside présente une forte inhibition, de 60 à 80 %, pour des concentrations allant de 1 mg/mL à 50 µg/mL.
L'undécyl-rhamnoside présente une activité comparable au pentyl rhamnoside dans la gamme de 20 µg/ml, ce qui lui confère une activité inflammatoire comparable.

### 4) Adhésion entre cellules endothéliales et lymphocytes

L'influence des alkyl-rhamnosides sur l'adhésion entre lymphocytes et cellules endothéliales non-activées a été évaluée, notamment la lignée de cellules endothéliales issues de la peau (HSkMEC). Ces cellules ont été mises en présence d'un fort activateur, le TNF-α.
L'adhésion est réalisée in-vitro en condition statique. Les cellules endothéliales sont ensemencées dans des puits afin d'obtenir une monocouche. Les cellules sont prétraitées pendant 5 heures en présence ou en absence d'alkyl-rhamnosides. Le dodécyl-rhamnoside nécessitant une teneur de 0,1 % volumique pour être soluble dans les milieux de culture, un témoin contenant 0,1 % de glycérol sera également analysé. En effet, le glycérol à 0,1 % stimule l'adhésion des lymphocytes sur HSkMEC (31 %) alors qu'il n'a pas d'effet sur les autres lignées de cellules endothéliales.

La suspension de lymphocytes marqués est à une concentration permettant d'obtenir un rapport de 5 lymphocytes pour une cellule endothéliale. L'adhésion est réalisée durant 30 minutes à température ambiante. Le marqueur se fixe irréversiblement aux lipides de la membrane plasmique des cellules sans affecter les propriétés biologiques de la membrane, ni la viabilité cellulaire.
L'adhésion des lymphocytes sur les cellules endothéliales est quantifiée par cytométrie de flux.
Les premiers tests d'adhésion sur les lignées de cellules endothéliales non activées ont donné les résultats suivants. Le pentyl-rhamnoside 1,1 mM induit une augmentation de l'adhésion des lymphocytes sur HPLNEC.B3 (37,9 %) et une très forte augmentation de l'adhésion sur HMLNEC par rapport au rhamnose (96,7 %). Il a un effet inhibiteur de l'adhésion des lymphocytes sur HSkMEC de 37,3 % par rapport au rhamnose. Il n'a pas ou très faiblement d'effet sur HAPEC et HBrMEC.
Le dodécyl-rhamnoside à 1,5 µM diminue l'adhésion des lymphocytes sur HSkMEC de 34,2 %. Il augmente cette adhésion sur HAPEC de 44,1 % et n'a pas d'effet sur HBrMEC, HPLNEC.B3 et HMLNEC.
Les résultats de l'adhésion entre lymphocytes et les cellules endothéliales activées, résumés dans le tableau ci-dessous (cf. tableau 5), sont issus du regroupement de trois expériences d'adhésion.

**Tableau 5 : Résultats de l'adhésion entre lymphocyte et cellules endothéliales (HSkMEC) en présence des dérivés rhamnosylés.**

| | **Milieu** | **TNF** | **Rapport Témoin/TNF** |
|---|---|---|---|
| Témoin | 1,000 | 2,730 | **2,7** |
| Rhamnose | 1,630 | 2,450 | **1,5** |
| Pentyl-rhamnose | 1,900 | 3,100 | **1,6** |
| Témoin glycérol | 2,200 | 3,200 | **1,45** |
| Dodécyl-rhamnose | 3,200 | 1,900 | **0,59** |

L'effet du dodécyl-rhamnoside à 1,5 µM est confirmé sur les cellules endothéliales avec une inhibition de l'adhésion de 63 %.

### Exemple 5: évaluation du potentiel irritant cutané sur épidermes reconstitués du pentyl rhamnoside

L'évaluation du potentiel irritant cutané sur épiderme reconstitué est une méthode alternative à l'expérimentation animale pour l'évaluation du potentiel irritant cutané sur épiderme reconstitué . Le principe est basé sur l'évaluation du potentiel irritant du produit testé par :
- Etude de la cytotoxicité par quantification de la libération de lactate deshydrogénase (LDH) et par réduction du MTT en sel de tétrazolium
- Etude des marqueurs de l'inflammation par quantification de la libération des interleukines IL 1α et IL8.

Les résultats sont résumés dans le tableau ci-dessous (cf. tableau 6)

**Tableau 6 : Résultats de l'évaluation du potentiel irritant cutané sur épidermes reconstitués du pentyl rhamnoside**

| | | Rh-C₅ Concentration 30% | Triton 4% |
|---|---|---|---|
| 24 heures | MTT % viabilité | 89,9 | 3,0 |
| d'application | Rapport LDH | 1,4 | 124,7 **I** |
| | Rapport IL_{1α} | 2,4 | 36,0 |
| | Rapport IL₈ | 5,2 | 3,7 |
| | | | |
| 72 heures | MTT % viabilité | 82,1 | 1,5 |
| d'application | Rapport LDH | 1,0 | 77,3 |
| | Rapport IL_{1α} | 2,0 | 7,2 |
| | Rapport IL₈ | 4,0 | 0,9 |
| Classification | | Légèrement irritant | Irritant |

Les résultats montrent que le pentyl rhamnoside, à une concentration de 30%, est faiblement irritant.

### Exemple 6: Etude du pouvoir sensibilisant par la méthode LLNA du pentyl rhamnoside

Les abréviations LLNA signifient Local lymph Node Assay, qui est une méthode alternative à l'expérimentation sur cobaye du pouvoir sensibilisant.
Ce test détermine le potentiel sensibilisant de la substance d'essai en mesurant la prolifération des lymphocytes dans les ganglions lymphatiques auriculaires. La prolifération des lymphocytes sera mesurée en déterminant l'incorporation de méthylthymidine tritiée.
Les résultats sont résumés dans le tableau ci-dessous (cf. tableau 7)

**Tableau 7 : Résultats de l'étude du pouvoir sensibilisant par la méthode LLNA du pentyl rhamnoside**

| Concentrations étudiées | Solvant | Concentration 1 : 3% | Concentration 2 : 15% | Concentration 3 : 30% | DNCB Dilution 0,25 % |
|---|---|---|---|---|---|
| DPM-BLANC | 13814,5 | 9921,5 | 8328,5 | 6378,5 | 125622,5 |
| DPM/Ganglion | 1726,8 | 1240,2 | 1041,1 | 797,3 | 15702,8 |
| RATIO | | 0,7 | 0,6 | 0,5 | 9,1 |
| Conclusion | | Non sensibilisant | Non sensibilisant | Non sensibilisant | Sensibilisant |

Dans le tableau 7, l'abréviation DPM signife « désintégration par minute », le blanc est la référence et le DNCB est le dinitrochlorobenzène qui sert de témoin sensibilisant. Les résultats montrent que le pentyl rhamnoside, même jusqu'à une concentration de 30%, n'est pas sensibilisant.

## Revendications

1. Composition comprenant au moins un monomère de rhamnose, dont la fonction hydroxyle anomérique est substituée par un radical alkoxy en C₅-C₁₂ pour le traitement cosmétique des peaux et/ou des muqueuses sensibles, irritées, intolérantes, à tendance allergique, âgées, soumises à un signal danger, présentant un trouble de la barrière cutanée, présentant des rougeurs cutanée ou présentant un déséquilibre immunologique non pathologique lié au vieillissement intrinsèque, extrinsèque ou hormonal.

2. Composition comprenant au moins un monomère de rhamnose, dont la fonction hydroxyle anomérique est substituée par un radical alkoxy en C₅-C₁₂ pour retarder le vieillissement naturel de la peau et/ou pour prévenir le vieillissement accéléré de la peau soumise aux agressions extérieures, notamment pour prévenir le vieillissement photo-induit de la peau.

3. Composition selon l'une quelconque des revendications 1 ou 2, **caractérisée en ce qu'**elle comprend 0,001 à 50% en poids dudit monomère.

## Claims

1. Composition comprising at least one rhamnose monomer, whose anomeric hydroxyl function is substituted by C₅-C₁₂ alkoxy radical for the cosmetic treatment of skins and/or mucous membranes that are sensitive, irritated, intolerant, of an allergic tendency, aged, subjected to a danger sign, exhibiting a disorder of the cutaneous barrier, exhibiting cutaneous redness or exhibiting a non-pathological immunological imbalance related to intrinsic, extrinsic or hormonal aging.

2. Composition comprising at least one rhamnose monomer, whose anomeric hydroxyl function is substituted by C₅-C₁₂ alkoxy radical to slow the natural aging of the skin and/or to prevent the accelerated aging of skin subjected to external attacks, in particular to prevent photo-induced aging of the skin.

3. The composition according to claim 1 or claim 2, wherein said composition contains from 0.001% to 50% by weight of said monomer.

## Patentansprüche

1. Zusammensetzung, umfassend mindestens ein Rhamnose-Monomer, dessen anomere Hydroxylfunktion durch einen C₅-C₁₂-Alkoxyrest substituiert ist, für die kosmetische Behandlung von Häute und/oder Schleimhäuten, die empfindlich, gereizt, intolerant sind, eine Allergieneigung aufweisen, gealtert sind, einem Gefährdungssignal ausgesetzt sind, eine Störung der Hautbarriere aufweisen, Hautrötungen aufweisen oder ein nicht-pathologisches immunologisches Ungleichgewicht, das mit der intrinsischen, durch äußere Einwirkungen verursachten oder hormonalen Alterung verbunden ist, aufweisen.

2. Zusammensetzung, umfassend mindestens ein Rhamnose-Monomer, dessen anomere Hydroxylfunktion durch einen C₅-C₁₂-Alkoxyrest substituiert ist, um die natürliche Alterung der Haut zu verzögern und/oder um die beschleunigte Alterung der Haut, die äußeren Angriffen ausgesetzt ist, zu verhüten, insbesondere um die durch Licht induzierte Alterung der Haut zu verhüten.

3. Zusammensetzung nach einem der Ansprüche 1 oder 2, **dadurch gekennzeichnet, dass** sie 0,001 bis 50 Gew.-% des Monomers umfasst.
